# EUROPEAN PATENT APPLICATION

(11) **EP 1 818 079 A1**
(43) Date of publication of application: **15.08.2007**
(21) Application number: 06009211.1
(22) Date of filing: 04.05.2006
(51) Int. Cl.: A62B 23/06

(54) **Nose filter**

(30) Priority: 14.02.2006 CN 200620003694
(71) Applicant: Chen, Chun-Hsien, Taipei (TW); Hsu, Chih-Chang, Taipei (TW)
(72) Inventor: Chen, Chun-Hsien, Taipei (TW); Hsu, Chih-Chang, Taipei (TW)
(74) Representative: Haft, von Puttkamer, Berngruber

(57) **Abstract**

The present invention relates to a nose filter, and more particularly to a nose filter to filter the dust or the allergen in the air. The nose filter mainly constitutes of two filter unit (1) and one connection unit (2), wherein the filter unit is positioned into the user nasal cavity to filter the dust or the allergen in the air. The connection unit is a U-shaped plastic, and then the two filter units are fixed to each end of the U-shaped connection unit.

## Description

### FIELD OF THE INVENTION

Around our daily environment, there are many tiny airborne pollute particles such as dust, pollen, spores, mold, fumes, and other allergens. People usually use face mask to block most airborne particles from being inhaled. However, the face mask is unable the complete fitting different people's facial features, it is hard to block most pollute particles by wearing a face mask. Meanwhile, in some public occasions, such as bank or restaurant, people wear a face mask will not be welcome. Because of the deterioration of the quality of environment air, the population of people who are suffering allergy is increased. Therefore, it is necessary to seek for a new filter device other than face mask for filtering airborne pollution particles and allergen.

Regarding seek for a new filter device other than face mask for filtering airborne pollution particles and allergen, Taiwan Pat. No. 00421029(issued on February 01, 2001) discloses a nose filter which is integrally formed with a pair of filter units connected with a connecting piece, wherein the filter unit is consist of two metal ring and an unwoven filter material. The nose filters are positioned in user's nasal cavity and, therefore, the filtering effect of nose filter will be better than face mask. The nose filters disclosed by TW 00421029 perform better function to filter airborne pollution particles and allergies than face masks. However, there are some drawbacks with using this nose filter. First, before using the nose filter, the user should fill and fix a filtering sheet, such as unwoven material or active carbon fiber, to the metal rings and cut out the excess portion of the filtering sheet by using scissors and after using the nose filter, the user should remove the filtering sheet from the metal ring and clean all component of the nose filter for next usage. The assembling process of nose filter is inconvenience and repeat use of the metal rings is unsanitary. Second, the nose filter used metal rings to fix the unwoven filter material and the connecting piece between metal rings is also made of metal material. Because of the metal ring is non-flexible, the different wearer should choice suitable sizes of metal rings to meet the different sizes of nostril. It is troublesome that using a over size metal rings will made wearer uncomfortable, but using under size metal rings will cause leakage and less filtering efficiency. Meanwhile, the visible metal connecting piece is not tasteful.

Following above mentioned patent, there are several patents in connection with new filter device for filtering airborne pollution particles and allergen. For example, Taiwan Pat. No. M270818(issued on July 21, 2005) discloses a nose plug filter, which comprising a hollow filter body and the filter body has an air tunnel with one-way valve and air inlet with a filtering sheet. This invention has some merits including high efficiency of filtering the pollution particles and allergen as well as easily breath. Unfortunately, the nose plug filter of this invention will be very expansive due to its complicated design results a high production cost and, additionally, not easy to use. Taiwan Pat. No. M273357(issued on August 21, 2005) discloses a nose mask, which comprising a nose-shaped mask with flexible material. The nose-shaped mask has at least one through hole and a filtering component fixed on the through hole. Because of the flexibility of nose-shaped mask, the nose mask of this invention can attach user's face more closely and result a higher filtering efficiency. Unfortunately, the look of the wearer's nose covered by a nose-shaped mask is very ugly. Taiwan Pat. No. M273354(issued on August 21, 2005) discloses a multifunction nose mask, which comprising a nose-shaped mask with flexible material and performing functions of anti-chilly and filtering airborne pollution. While the filtering efficiency of the nose mask is very good, the look is also very ugly.

### SUMMARY OF THE INVENTION

It is an object of the present invention to provide a nose filter, which has circular filtering material to filter dust, tiny pollution particles and allergen. The nose filter has high filtering efficiency, but with no ugly look of filters like the nose mask.

It is a further object of the present invention to provide a nose filter produced by a very simple assembling that may effectively reduce the production cost. The nose filter of present invention is convenient and hygienic to user because it is not expensive and all materials are not necessary to reuse.

It is a further object of the present invention to provide a nose filter has a flexible disk-shaped filter with several radial slits. The radial slits let the disk-shaped filter more flexible and better distortion. Therefore, it has an advantage of a single size disk-shaped filter may be matched to all user with different sizes of nasal cavity and results more comfortable and higher filtering efficient.

It is another object of the present invention is to provide a nose filter has a transparent U-shaped connection unit. The wearer may feel comfortable with good look because the exposed U-shaped connection unit of this nose filter is invisible.

### BRIEF DESCRIPTION OF DRAWINGS

FIG. 1 is the schematic drawing showing the component combination of this invention nose filter.
FIG. 2 is the schematic drawing showing another component combination of this invention nose filter.
FIG. 3 is the invention nose filter.
FIG. 4 is another combination of this inventions nose filter.

### DETAILED DESCRIPTION OF THE INVENTION

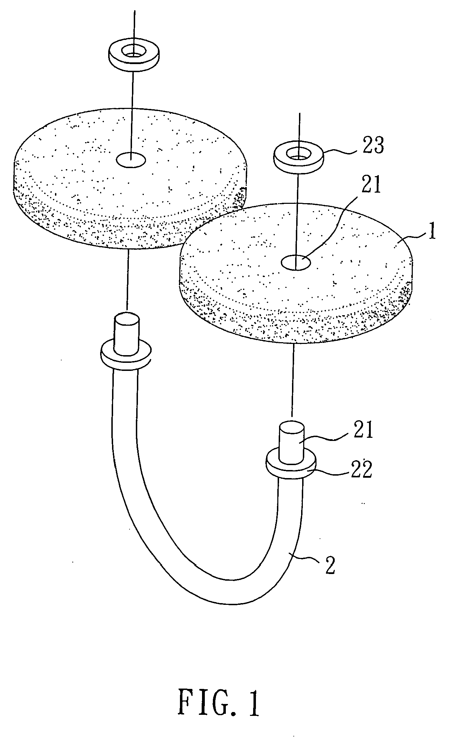

The present invention relates to a nose filter, and more particularly to a nose filter to filter the dust or the allergen in the air. The nose filter mainly constitutes of two filter units and one connection unit, wherein the filter unit is positioned into the user nasal cavity to filter the dust, general pollutants or the allergen in the air. The connection unit is a U-shaped plastic, and then the two filter units are fixed to the each end of the U-shaped connection unit. FIGS.1 illustrates one embodiment of a nose filter with disk-shaped filter unit, the filter unit 1 is a flexible disk-shaped filter material having suitable thickness and a central through hole 11, and the connection unit 2 is a U-shaped element. The end of the U-shaped connection unit 21 penetrated the central through hole 11 of the filter unit 1 and the two disk-shaped filter units 1 are fixed to each end of the U-shaped connection unit 21.

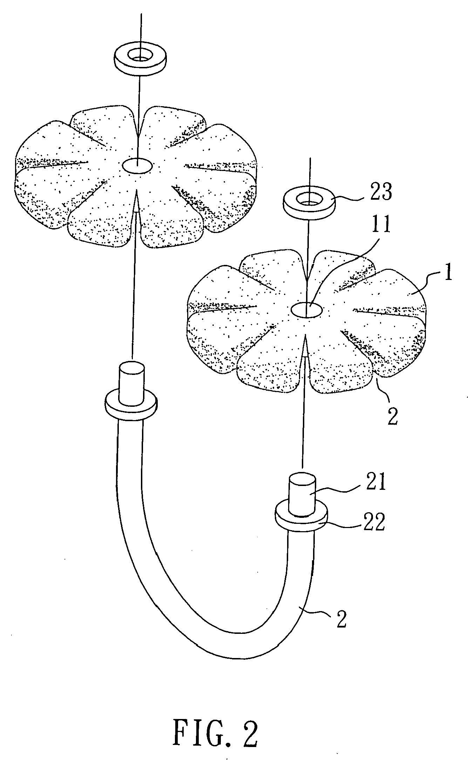

The filter materials of the air filter unit of this invention are made from unwoven filter material. The unwoven filter materials are already widely used in the medical application and medical facility. The thickness of the air filter unit of this invention is compromised between air permeability and filter efficiency of the unwoven filter material. The exemplary of the filter unit 1, the flexible disk-shaped filter material may deform slightly to match for the user nasal cavity. Typically, the suitable sizes of the filter unit of this invention are always larger than the user nasal cavity, so that the disk-shaped filter material may fill closely the user nasal cavity, therefore, the air filter unit of this invention may be a disk-shaped, a ball-shaped or an ellipsoid-shaped filter material. For the proposal to the convenient and comfortable, the disk-shaped filter unit 1 may have several radial slits 12. FIG. 2 illustrates an alternative configuration for the disk-shaped filter unit 1 with several radial slits 12, which result the disk-shaped filter unit 1 more flexible. After the disk-shaped filter unit 1 with radial slits 12 positioned into user nasal cavity, the slit 12 will be closed up due to slight distortion of the filter material, therefore, the disk-shaped filter unit 1 can be matched to all user with different size of nasal cavity, and results more comfortable and higher filtering efficient. Additionally, it will be more comfortable that modifying the periphery of the radial slits 12 of disk-shaped filter unit 1 to smooth the edge of filter unit.

The connection between two air filter units is a U-shaped connection unit 2, wherein the U-shaped connection unit 2 has a stop ring 22 near the end 21. The end of the U-shaped connection unit 2 penetrated the through hole 11 of disk-shaped filter unit 1, a top ring 23 is fixed to said end 21, and the disk-shaped filter unit 1 is fixed between the stop ring 22 and the top ring 23.

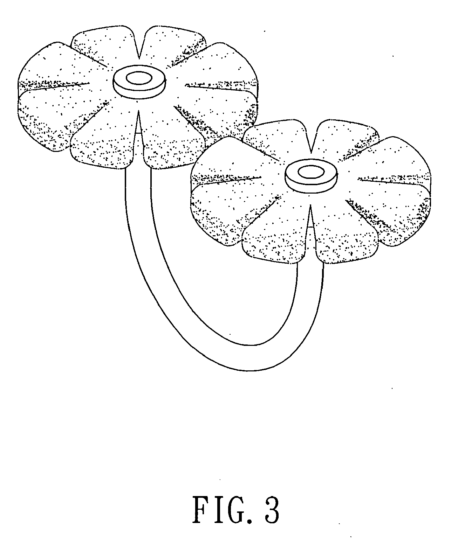

As shown in FIG. 3, the assembly of the nose filter of this invention is very simple and the production cost of the nose filter is very low. Therefore, the nose filter is a disposable device and all materials are not necessary to reuse, because the nose filter is inexpensive. The advantage is user need not assemble the nose filter by himself, nor disassemble and clean the used nose filter.

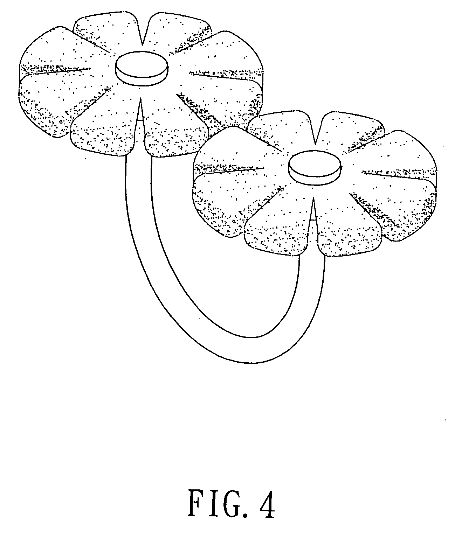

The assembly of two air filter units and one U-shaped connection unit of above-mentioned nose filter is only one exemplary disclosure of this invention. Alternatively, the end of the U-shaped connection unit penetrated the through hole of disk-shaped filter unit, and then said end is flatten by a method of thermal pressing, and the disk-shaped filter unit is fixed between the stop ring and the flatten end, as shown in Fig 4. This assembly may simplify the production and lower the cost of the nose filter, and particularly suitable for disposable nose filter.

The U-shaped connection unit of this invention is a strip of transparent plastic, which is flexible and invisible. The wearer will feel more comfortable because the transparent property may conceal the outward appearance of U-shaped connection unit.

While particular forms of the invention have been illustrated and described, it will be apparent that various modifications can be made without departing from the spirit and scope of the invention.

## Claims

1. A nose filter, which is positioned in user nasal cavity used for filtering general pollutants or allergens in air, comprising of two air filter units and one U-shaped connection unit, wherein said two air filter units are fixed to each end of the U-shaped connection unit.

2. A nose filter of claim 1 wherein the air filter unit is made from unwoven filter material.

3. A nose filter of claim 1 wherein the air filter unit is a disk-shaped, a ball-shaped or an ellipsoid-shaped filter material.

4. A nose filter of claim 1 wherein the air filter unit is a flexible disk-shaped filter material having suitable thickness and a central through hole, wherein the end of the U-shaped connection unit is penetrated into said central through hole, and the two air filter units are fixed to each end of the U-shaped connection unit.

5. A nose filter of claim 1 wherein the disk-shaped filter unit having several radial slits.

6. A nose filter of claim 1 wherein the U-shaped connection unit is a strip of transparent plastic.

7. A nose filter of claim 1 wherein the U-shaped connection unit having a stop ring near the end.

8. A nose filter of claim 7 wherein the end of the U-shaped connection unit penetrated the through hole of disk-shaped filter unit, a top ring is fixed to said end, and the disk-shaped filter unit is fixed between the stop ring and the top ring.

9. A nose filter of claim 7 wherein the end of the U-shaped connection unit penetrated the through hole of disk-shaped filter unit, and then said end is flatten by a method of thermal pressing, and the disk-shaped filter unit is fixed between the stop ring and the flatten end.
